# NEUE EUROPÄISCHE PATENTSCHRIFT

(11) **EP 0 274 086 B2**
(45) Veröffentlichungstag und Bekanntmachung des Hinweises auf die Entscheidung über den Einspruch: **26.11.1997**
(45) Hinweis auf die Patenterteilung: 28.08.1991
(21) Anmeldenummer: 87118483.4
(22) Anmeldetag: 14.12.1987
(51) Int. Cl.: A61K 7/11

(54) **Haarfestlegemittel**
Hair fixing composition
Composition de fixation des cheveux

(30) Priorität: 22.12.1986 DE 3643897
(43) Veröffentlichungstag der Anmeldung: 13.07.1988
(73) Patentinhaber: Henkel Kommanditgesellschaft auf Aktien, 40191 Düsseldorf (DE)
(72) Erfinder: Giede, Karl, D-4010 Hilden (DE); Höffkes, Horst, Dr., D-4000 Düsseldorf-Hellerhof (DE)

(56) Entgegenhaltungen:
- FR-A- 2 224 497
- GB-A- 1 595 649
- US-A- 3 726 288
- US-A- 3 810 977
- K. Schrader, Grundlagen und Rezepturen des Kosmetika (1979) S. 56, 88, 137, 157-158, 480-485.
- Lexikon der Hilfstoffe für Pharmazie, Kosmetik und angrenzende Gebiete, Dr. H. Fiedler (Hrsg.) (1981) Einträge: Resyn 28-1310, 28-2930, 2-Amino-2-methyl-1,3-propandiol, 2-Amino-2-methyl-1-propanol
- Produktinformation "National Starch" zu "National 28-2930" vom 25.1.90

## Beschreibung

Gegenstand der Erfindung sind Haartestlegemittel in Form von wäßrigen oder wäßrig-alkoholischen Zubereitungen mit einem Gehalt an durch Neutralisation gelösten, carboxylgruppenhaltigen Polymeren.

Ursprünglich wurden Haarsprays in Form von alkoholischen Lösungen wasserunlöslicher Harze formuliert. Diese Produkte hatten den Nachteil, daß sie das Haar klebrig machten und schwer wieder aus dem Haar entfernt werden konnten. Einen merklichen Fortschritt stellte daher die Verwendung von carboxylgruppenhaltigen Harzen dar, die durch Neutralisation in Wasser oder wäßrig-alkoholischer Lösung löslich gemacht werden konnten und die sich später wesentlich leichter wieder aus dem Haar entfernen lassen. Über solche Harze und deren Anwendung wird in Cosmetics & Toileteries Vol. 94 (April 1979), Seiten 75 bis 76 berichtet.

Die günstigsten Anwendungseigenschatten weisen solche carobxylgruppenhaltigen Harze auf, wenn die Carboxylgruppen nur zu etwa 50 bis 90 Mol% neutralisiert sind. Sie sind dann zwar schon gut aus dem Haar auswaschbar, bewirken aber noch eine hohe Flexibilität und Curl-Retention des Haars. Leider benötigt man zur Herstellung klargelöster Formulierungen wäßrigen Alkohol mit einem relativ hohen Alkoholgehalt, was mit erheblichen Kosten verbunden ist. Vollständig neutralisierte Harze sind zwar bereits in Wasser oder wäßrig-alkoholischen Lösungen mit niedrigem Alkoholgehalt gut löslich, zeigen jedoch deutlich schlechtere Anwendungseigenschatten: Es bilden sich brüchige Filme am Haar, die beim Durchkämmen stauben und die nur eine verringerte Curl-Retention aufweisen.

Es wurde nun gefunden, daß man diese Probleme weitgehend ausschalten und Haarfestlegemittel auf wäßriger Basis oder in Form wäßrig alkoholischer Lösungen mit niedrigem Alkoholgehalt formulieren kann, die bei der Anwendung sehr gute Filmelastizität und Curl-Retentionswerte zeigen, wenn man die carboxylgruppenhaltigen Filbildner bis zu dem Neutralisationsgrad, der die optimalen Anwendungseigenschaften am Haar bewirkt, mit Alkanolaminen und darüber hinaus bis zum Erreichen einer vollständigen, d. h. klaren Lösung in Wasser oder verdünntem Alkohol mit Ammoniak neutralisiert.

Gegenstand der Erfindung sind daher Haartestlegemittel in Form von wäßrigen oder wäßrig-alkohlischen Zubereitungen mit einem Gehalt an durch Neutralisation gelösten, carboxylgruppenhaltigen Polymeren, wobei der Gehalt an niederen Alkoholen maximal 15 Gew.-% beträgt. dadurch gekennzeichnet, daß 50 bis 90 Mol% der Carboxylgruppen des Polymeren mit Alkanolaminen mit 2 bis 10 C-Atomen und zu 10 bis 50 Mol% mit Ammoniak neutralisiert sind, und die Mittel weiterhin ein wasserlösliches kationisches Polymer enthalten.

Haarfestlegemittel im Sinne der vorliegenden Erfindung sind alle Zubereitungen, die nach der Anwendung auf dem Haar verbleiben und dort eine festigende, die Frisur haltbarmachende Wirkung entfalten; hierzu gehören vor allem flüssige Haartestiger, auch aerosolverpackte Schaumfestiger, Gelfestiger und Haarsprays.

Unter wäßrigen und wäßrig-alkoholischen Zubereitungen werden Zubereitungen verstanden werden, die frei von niederen Alkoholen sind oder niedere Alkohole in untergeordneten Mengen von 0 bis 15 Gew.-% enthalten. Niedere Alkohole, die für diese Zwecke gebräuchlich sind, sind vor allem Ethanol und Isopropanol.

Geeignete carboxylgruppenhaltige Polymere für die Verwendung in erfindungsgemäßen Haarfestlegemitteln sind vor allem Mischpolymerisate aus mindestens einer ungesättigten Monocarbonsäure und mindestens einem Vinyl- oder Vinylidenmonomeren. Als Monocarbonsäure-Monomere kommen z. B. Acrylsäure, Methacrylsäure, Crotonsäure, Allylessigsäure, 2-Allyloxypropionsäure, Betabenzoylacrylsäure,2-Vinylpropionsäure oder Vinylessigsäure in Frage. Als Comonomere können z. B. Styrol, Acryl- und Methacrylsäureester, Acrylamid, Methacrylamid, Diester der Malein- und Fumarsäure. Vinylether sowie gegebenenfalls auch kationische Monomere wie z. B. Tert.-Butylaminoethylmethacrylat, Dimethyl- und diethylaminoethylmethacrylat.

Bevorzugt werden Mischpolymerisate aus Acrylsäure, Methacrylsäure und/oder Crotonsäure und Comonomeren vom Typ der Vinylester, der Acryl- und Methacrylsäureester und/oder der Acryl- und/oder Methacrylamide mit einer Acidität von 0,7 bis 2,1 mval/g eingesetzt.

Die genannten carboxylgruppenhaltigen Polymeren sind in den erfindungsgemäßen Haarfestlegemitteln bevorzugt in einer Menge von 1 bis 5 Gew.-% enthalten.

Besonders gut geeignete carboxylgruppenhaltige Polymere sind z. B. Copolymere aus Crotonsäure und Vinylacetat (Molverhältnis 10 : 90). Produkte dieser Art sind unter der Handelsbezeichnung Resyn^{(R)}28-1310 (National Starch and Chem. Corp.) oder Luviset^{(R)}CA66 (BASF) erhältlich. Weitere sehr gut geeignete carboxylgruppenhaltige Polymere sind z. B. Terpolymerisate aus Vinylacetat, Crotonsäure und einen Vinylester einer verzweigten Carbonsäure, z. B. von 2,2-Dimethyloctansäure. Solche Terpolymerisate sind z. B. aus DE-AS 17 45 208 bekannt und unter der Bezeichnung Resyn^{(R)}28-2930 (National Starch and Chem. Corp.) im Handel erhältlich.

Auch amphotere Polymerisate, die sowohl Carboxylgruppen als auch Aminogruppen aufweisen, sind geeignet, z. B. das Mischpolymerisat aus N-Octyl-acrylamid, Methylmethacrylat, Hydroxypropylmethacrylat, Acrylsäure und tert.-Butylaminoethylmethacrylat, welches unter der Bezeichnung Amphomer^{(R)}(National Starch and Chem. Corp.) im Handel erhältlich ist.

Als Alkanolamine mit 2 bis 10 C-Atomen werden für die Neutralisation z. B. 2-Amino-2-methyl-propan-1,3-diol, 2-Amino-2-ethyl-propan-1,3-diol, 2-Amino-2-methyl-1-propanol, Tris-(hydroxymethyl)-aminomethan, 1-Amino-2-methyl-propan-2-ol, 3-Aminopentan-2-ol, 2-Amino-1-phenyl-butan-1-ol, 2-Dimethylamino-2-methyl-propan-1-ol, N-(2-hydroxyethyl)-2-methyl-propylen-1,2-diamin oder Tris-(hydroxymethyl)-dimethylaminomethan verwendet. Abhängig von der Art und Acidität des verwendeten carboxylgruppenhaltigen Polymeren und der Art des verwendeten Alkanolamins wird durch Neutralisation von 50 bis 90 Mol% der vorhandenen Carboxylgruppen mit dem Alkanolamin eine hohe Elastizität der mit dem Polymerisat behandelten Haare aber noch keine klare Löslichkeit in Wasser oder in wäßrig-alkoholischen Zubereitungen mit bis zu 15 Gew.-% des niederen Alkohols erreicht. Zur weiteren Neutralisation bis zur völligen Lösung des Harzes wird daher Ammoniak in einer Menge von 10 bis 50 Mol% der vorhandenen Carboxylgruppen verwendet. Beim Trocknen des Films auf dem Haar verdunstet offenbar ein Teil des Ammoniaks und hinterläßt das Harz in der teilneutralisierten Form, in der es die optimalen Anwendungseigenschaften aufweist.

Weiterhin enthalten die erfindungsgemäßen Haarfestlegemittel zwingend ein wasserlösliches kationisches Polymer.

Diese kationaktiven wasserlöslichen Polymeren mit haaravierender Wirkung, bevorzugt im Molekulargewichtsbereich von 1 000 bis 3 000 000, enthalten entweder in der Polymerkette freie oder alkylsubstituierte Aminogruppen oder quartäre Ammoniumgruppen oder tragen an die Polymerkette direkt oder über Zwischenglieder gebundene, sekundäre oder tertiäre Aminogruppen oder quartäre Ammoniumgruppen und sind zu wenigstens 0,5 Gew.-% in Wasser bei 20°C löslich. Die Aminogruppen oder die quartären Ammoniumgruppen können auch Glieder von 5- oder 6gliedrigen Ringsystemen sein, z. B. von Morpholin, Piperidin-, Piperazin- oder Imidazol-Ringen. Zahlreiche wasserlösliche kationische Polymere sind in der Literatur beschrieben und im Handel erhältlich. Bevorzugt werden als kationische Polymere solche mit quartären Ammoniumgruppen eingesetzt. Besonders geeignete kationische Polymere sind z. B. die aus der US-PS 3,910,862 bekannten und unter der Handelsbezeichnung GAFQUAT^{(R)}734, 750 und 755 erhältlichen quaternierten Vinylpyrrolidon-Dialkylaminoalkylmethacrylat Copolymerisate, die aus US-PS 3,632,559 bekannten und z. B. unter der Handelsbezeichnung CARTARETINE^{(R)}F4 erhältlichen Copolymeren aus Adipinsäure und Dimethylaminohydroxypropyl-diethylentriamin, die aus US-PS 4,157,388 bekannten und z. B. unter der Handelsbezeichnung MIRAPOL^{(R)}A15 erhältlichen quartären polymeren Harnstoffderivate und die aus US-PS 3,912,808 bekannten und unter der Handelsbezeichnung MERQUAT^{(R)}-100 oder 550 (Quaternium 41) erhältlichen Dialkyl-diallylammonium-Polymerisate. Von diesen kationischen Polymeren sind bevorzugt 0,1 bis 1,0 Gew.-% enthalten.

Außer den carboxylgruppenhaltigen Polymeren, die in der erfindungsgemäßen Weise neutralisiert und in der wäßrigen oder wäßrig-alkoholischen Zubereitungen gelöst sind, können die erfindungsgemäßen Haarfestlegemittel noch weitere, für solche Formulierungen an sich bekannte Zusätze und Hilfsmittel enthalten:
- Kationische oberflächenaktive Stoffe mit antistatischer Wirkung, bevorzugt solche vom Typ der quartären Ammoniumverbindungen. Dies sind bevorzugt Verbindungen, die eine quartäre Ammoniumgruppe in Form des Chlorids, Bromids, Sulfats, Phosphats, Methosulfats oder Ethosulfats tragen, die mit ein oder zwei langkettigen, bevorzugt linearen Alkyl-, Hydroxyalkyl- oder Alkyl-(poly)-oxyethylgruppen mit 10 bis 22 C-Atomen in der Alkyl- oder Hydroxyalkylgruppe und einer oder zwei Alkyl-. Hydroxyalkyl- oder Polyhydroxyalkylgruppen mit 1 bis 4 C-Atomen in der Alkylgruppe und ggf. einer Benzylgruppe substituiert sind. Geeignet sind auch Alkylpyridinium-Salze und Alkyl-Imidazoliniumsalze mit 10 bis 22 C-Atomen in der Alkylgruppe. Beispiele für geeignete quartäre Ammoniumverbindungen sind Cetyltrimethylammoniumchlorid, Lauryldimethylbenzyl-ammoniumchlorid. Stearyl-tris-(polyoxyethyl)-ammonium-phosphat, Cetylpyridiniumchlorid, Distearyl-dimethylammoniumchlorid oder 2-Hydroxyhexadecyl-2-hydroxyethyl-dimethyl-ammoniumchlorid. Von diesen oberflächenaktiven, quartären Ammoniumverbindungen sind bevorzugt 0,05 bis 1,0 Gew.-% in den erfindungsgemäßen Haarfestlegemitteln enthalten.
- Verdickungsmittel vom Typ der nichtionogenen, wasserlöslichen Polymeren. z. B. Hydroxyethylcellulose, Hydroxypropylcellulose, Methylcellulose, Guar-Gum, Xanthan-Gum, Polyvinylpyrrolidon, Polyethylenglykole, Polyacrylamid und andere bekannte natürliche und synthetische wasserlösliche Polymere. Diese dienen bevorzugt zur Stabilisierung des Schaumes in Schaumaerosolzubereitungen. Bevorzugt sind 0,1 bis 1,0 Gew.-% an nichtionischen, wasserlöslichen polymeren Verdickungsmitteln enthalten.
Eine weitere bevorzugte Ausführungsform besteht darin, daß man die Haartestlegemittel als schäumende Flüssigkeit formuliert und mit der erforderlichen Menge an verflüssigten Gasen, z. B. von Propan-Butan-Gemischen, Fluorkohlenwasserstofftreibmitteln, Dimethylether oder Mischungen dieser Treibmittel mit CO₂ und/oder N₂O (Distickstoffmonoxid) als Schaumaerosole zubereitet.

Neben den schon genannten obligatorischen und bevorzugt vorhandenen fakultativen Bestandteilen können die erfindungsgemäßen Haartestlegemittel noch weitere, für solche Zubereitungen übliche Hilfsmittel in untergeordneten Mengen enthalten. Dies sind in erster Linie
- kosmetische Ölkomponenten
- Duftstoffe
- nichtionogene Emulgatoren oder Lösungsvermittler für Duftstoffe oder Ölkomponenten
- Farbstoffe
- Trübungs- und Perlglanzmittel
- Konservierungsstoffe
- Lichtschutzmittel
- wasserlösliche Proteine, Proteinhydrolysate oder Proteinderivate
- Vitamine, Kräuterextrakte, Panthenol

## Patentansprüche

1. Haarfestlegemittel in Form Von wäßrigen oder wäßrig-alkoholischen Zubereitungen mit einem Gehalt an durch Neutralisation gelösten, carboxylgruppenhaltigen Polymeren wobei der Gehalt an niederen Alkoholen maximal 15 Gew % beträgt, dadurch gekennzeichnet, daß 50 bis 90 Mol% der Carboxylgruppen des Polymeren mit Alkanolaminen mit 2 bis 10 C-Atomen und 10 bis 50 Mol% mit Ammoniak neutralisiert sind, und die Mittel weiterhin ein wasserlösliches kationisches Polymer enthalten.

2. Haarfestlegemittel nach Anspruch 1, dadurch gekennzeichnet, daß 0 bis 15 Gew.-% Ethanol oder Isopropanol enthalten ist.

3. Haarfestlegemittel nach den Ansprüchen 1 oder 2, dadurch gekennzeichnet, daß als carobxylgruppenhaltige Polymere Mischpolymerisate aus Acrylsäure, Methacrylsäure und/oder Crotonsäure und Comonomeren vom Typ der Vinylester, der Acryl- und Methacrylsäureester und/oder der Acryl- und Methacrylamide mit einer Acidität von 0,7 bis 2,1 mval/g enthalten sind.

4. Haarfestlegemittel nach den Ansprüchen 1 bis 3, dadurch gekennzeichnet, daß das carboxylgruppenhaltige Polymere in einer Menge von 1 bis 5 Gew.-%, bezogen auf die gesamte Zusammensetzung, enthalten ist.

5. Haarfestlegemittel nach den Ansprüchen 1 bis 4, dadurch gekennzeichnet, daß das wasserlösliche kationische Polymere in Mengen von 0,1 bis 1,0 Gew.-% enthalten ist.

6. Haarfestlegemittel nach den Ansprüchen 1 bis 5, dadurch gekennzeichnet, daß als zusätzliches Hilfsmittel 0,05 bis 1,0 Gew.-% einer oberflächenaktiven quartären Ammoniumverbindung enthalten ist.

7. Haarfestlegemittel nach den Ansprüchen 1 bis 6, dadurch gekennzeichnet, daß als zusätzliches Hilfsmittel 0,1 bis 1,0 Gew.-% eines nichtionischen, wasserlöslichen, polymeren Verdickungsmittels enthalten ist.

## Claims

1. Hair setting preparations in the form of aqueous or aqueous/alcoholic preparations containing carboxy-functional polymers dissolved by neutralisation, wherein the content of lower alcohols is at most 15 wt.%, characterised in that 50 to 90 mol.% of the carboxyl groups of the polymer are neutralised with alkanolamines having 2 to 10 C atoms and 10 to 50 mol.% with ammonia and the preparations furthermore contain a water-soluble cationic polymer.

2. Hair setting preparations according to claim 1, characterised in that 0 to 15 wt.% of ethanol or isopropanol is present.

3. Hair setting preparations according to claims 1 or 2, characterised in that copolymers prepared from acrylic acid, methacrylic acid and/or crotonic acid and comonomers of the vinyl ester, acrylic and methacrylic acid ester and/or acrylamide and methacrylamide type having an acidity of 0.7 to 2.1 mval/g are present as the carboxy-functional polymers.

4. Hair setting preparations according to claims 1 to 3, characterised in that the carboxy-functional polymer is present in a quantity of 1 to 5 wt.%, relative to the entire composition.

5. Hair setting preparations according to claims 1 to 4, characterised in that the water-soluble cationic polymer is present in quantities of 0.1 to 1.0 wt.%.

6. Hair setting preparations according to claims 1 to 5, characterised in that 0.05 to 1.0 wt.% of a surface-active quaternary ammonium compound is present as an additional auxiliary substance.

7. Hair setting preparations according to claims 1 to 6, characterised in that 0.1 to 1.0 wt.% of a nonionic, water-soluble polymeric thickener is present as an additional auxiliary substance.

## Revendications

1. Compositions de fixation des cheveux sous la forme de préparations aqueuses ou aqueuses-alcooliques à base de polymères renfermant des groupes carboxyle, dissous par neutralisation, la concentration en alcools inférieurs s'élevant au maximum à 15 % en poids, caractérisées en ce que les groupes carboxyle du polymère sont neutralisés à 50 à 90 moles % par des amino-alcools comportant 2 à 10 atomes de C, et à 10 à 50 moles % par de l'ammoniac, et en ce que les produits renferment en outre un polymère cationique soluble dans l'eau.

2. Compositions de fixation des cheveux selon la revendication 1, caractérisées en ce quelles renferment 0 à 15 % en poids d'éthanol ou d'isopropanol.

3. Compositions de fixation des cheveux selon la revendication 1 ou 2, caractérisées en ce qu'elles contiennent comme polymères contenant des groupes carboxyle, des copolymères d'acide acrylique, d'acide méthacrylique et/ou d'acide crotonique et de comonomères du type des esters vinyliques, des esters acryliques et méthacryliques et/ou des acryl- et méthacrylamides, présentant une acidité de 0,7 à 2,1 mval/g.

4. Compositions de fixation des cheveux selon les revendications 1 à 3, caractérisées en ce que les polymères renfermant des groupes carboxyle sont contenus dans une proportion allant de 1 à 5 % en poids par rapport à la totalité de la composition.

5. Compositions de fixation des cheveux selon les revendications 1 à 4, caractérisées en ce que le polymère cationique soluble dans l'eau est contenu en proportions de 0,1 à 1,0 % en poids.

6. Compositions de fixation des cheveux selon les revendications 1 à 5, caractérisées en ce qu'elles contiennent comme adjuvant supplémentaire, 0,05 à 1,0 % en poids d'un composé d'ammonium quaternaire tensioactif.

7. Compositions de fixation des cheveux selon les revendications 1 à 6, caractérisées en ce qu'elles contiennent comme adjuvant supplémentaire, 0,1 à 1,0 % en poids d'un épaississant polymère, non ionique et soluble dans l'eau.
